# EUROPEAN PATENT SPECIFICATION

(11) **EP 0 550 842 B1**
(45) Date of publication and mention of the grant of the patent: **23.10.1996**
(21) Application number: 92121017.5
(22) Date of filing: 09.12.1992
(51) Int. Cl.: A61F 5/14

(54) **Adjustable orthotic**
Orthotische einstellbare Einlage
Insert orthotétique ajustable

(30) Priority: 10.12.1991 US 804423
(43) Date of publication of application: 14.07.1993
(73) Proprietor: Smith, Leland R., Foster City, California 94404 (US)
(72) Inventor: Smith, Leland R., Foster City, California 94404 (US)
(74) Representative: Patentanwälte Ruff, Beier, Schöndorf und Mütschele

(56) References cited:
- WO-A-89/01745
- DE-C- 279 735
- GB-A- 620 329
- GB-A- 2 124 473
- GB-A- 2 193 426
- US-A- 4 333 472
- US-A- 4 715 131

## Description

### Field of the Invention

This application relates to methods for forming orthopedic devices for use with shoes and other footwear, and to methods for forming such orthopedic devices.

### Background of the Invention

Orthopedic devices for the feet (hereinafter sometimes called "orthotics") are well known, and have been used by laypersons and podiatrists for many years. Orthotics of this type range from a simple arch support to a custom formed support for the foot. It has been estimated that 50% of the population could benefit from some form of shoe orthopedic device to improve support and balance for the foot.

Since the feet are the foundation on which the rest of the body is supported, foot misalignment can result in many forms of discomfort for the patient. Symptoms which have been known to develop from such misalignment are plantar fasciitis, hammertoes, bunions, achilles tendonitis, and others. Misalignment can also cause or exacerbate knee, hip or back problems.

In an ideal situation, orthotics can cure two forms of misalignment. First, the orthotic should match the sole of the foot to the ground and, second, the orthotic should bring the remainder of the body into proper alignment with the foot. It will be appreciated that the feet, legs and upper body represent a closed kinetic chain in which a change at any one point can affect the remaining points. Obtaining proper alignment of the foot with the ground can be simplifed to "bringing the ground up to meet the foot", while obtaining proper alignment of the foot with the rest of the body involves adjusting the relative position of the foot to the legs.

Many pre-formed orthotics are available to remedy a variety of patient maladies relating to posture, stance and gait. Most such pre-formed orthotics are formed of a polymeric material and sold in a variety of sizes and shapes, with the expectation that one or another of the pre-cast shapes will be close enough to the patient's needs that the orthotic will be acceptable.

However, in most such instances, the fit -- and thus the amount of improvement -- provided by the mass produced orthotic is far less than perfect. Typical problems involve the conformity of the orthotic to the sole of the foot, which affects the uniformity of support, and well as the amount of correction provided. Since only a few sizes of mass produced orthotics can be justified from an economic standpoint, this requires the patient to accept a significant amount of compromise in comfort and fit, and leads many patients to abandon use of the pre-formed orthotic.

Custom fitted orthotics are also available, but typically are very expensive. In the conventional approach, a custom orthotic is constructed by starting with a complete cast of the foot. Such custom orthotics are frequently, though not always, done under the supervision of a podiatrist. The impression which results from the rusting is then sent to a laboratory where the custom formed orthotic is developed from the impression. As a result, the custom orthotic typically conforms to the sole of the patient's foot much better than a mass produced orthotic. Such custom orthotics, however, have two serious limitations. While the fit is much better in most instances, if an error is made either in making the initial casting, or making the orthotic from the casting, the resulting orthotic is substantially useless. Second, and often more important, changes in the patient's physiology require that the custom orthotic be recast. This latter limitation with the existing art can also affect patients using mass produced orthotics, and is particularly applicable to children. Children typically outgrow custom inlays in approximately one year. Given the expense of such custom orthotics, the economic burden can become substantial for either type of recasting.

In order to correct specific abnormalities of the human foot, the use of separate movable wedge-shaped cushion pads is known (US-A-4 333 472). These pads made of a resilient foam material are placed individually in the inside of the shoe and affixed.

It is further known, to use shoe inserts in the form of a pad shaped to provide an arch support of the foot. This insert is not able to correct the alignment between the foot and the ground.

An orthotic according to the pre-characterising portion of claim 1 is known from DE-C-279735, which discloses a heel-bone support device having a cap made from springy metal which takes up the heel-bone. At the bottom of the cap is a flap. Between the flap and the bottom of the cap a wedge can be inserted.

Further an orthotic according to the pre-characterising portion of claim 2 is known WO-A-8 901 745. This orthotic corresponds to a foot bed sole conform to the shape of the human foot. This orthotic is inserted into a sheath made of flexible material which is then affixed to the sole of the shoe.

### Summary of the Invention

The aim of the present invention is to overcome many of the limitations of the prior art, and provides nearly all of the benefits of custom orthotics, while at the same time permitting adjustment of the orthotic after initial construction.

The present invention provides a fully custom, adjustable orthotic specially fitted to the patient, yet capable of being adjusted to ensure continued proper fit and support, and may be configured as a footsole to be placed in a shoe as defined in appended claim 1, or as part of the sole of a conventional shoe as defined in appended claim 2.

In particular, a pre-existing footsole portion of an orthotic formed of polyethylene terephthalate or other acceptable polymer has included in the bottom thereof attachment means. The posts or wedges, which may vary in configuration and amount of correction, include on the top thereof an attachment device capable of mating with the attachment device on the underside of the footsole portion. When attached, the post and footsole become substantially an integrated orthotic.

The invention provides devices for instances, where both the foreward portion of the foot and the heel need alignment correction. In such instances, the orthotic of the present invention includes multiple attachment devices to permit correction of alignment at both locations.

The attachment device may use any of a variety of techniques, including but not limited to interlocking dimples, Velcro™, **bolts, interlocking geometries** and dowels. The primary requirement of such attachment devices is that they remain firmly attached under the stress of walking and running, including remaining attached during the flexing of the orthotic which occurs with such activities.

By replacing one wedge with others having different correction factors, such as different wedge angles, an adjustable orthotic is provided which can be specially fitted to the patient. As a result, improved fit and support can be provided. For convenience, different colors may be used for different correction factors.

In addition, in the event the patient's physiology changes, the orthotic of the present invention can be modified readily to provide continued excellent support, thus substantially reducing the economic burden associated with both custom and mass produced orthotics. This can be particularly helpful for children. Additionally, the lower cost of the present device will make correction of gait problems in children much more accessible to a significant segment of the population.

Still further, the adjustable orthotic of the present invention may be configured as part of the sole of an otherwise conventional shoe. In such an arrangement, the sole of the shoe may be specially configured to permit detachable forefoot and heel portions to be added and adjusted in a manner otherwise similar to the orthotic.

There has therefore been a need for an orthotic which can be adjusted readily by a podiatrist to provide a substantially custom correction of a patient problem.

In addition, there has been a need for an orthotic which can be readily modified by altering the amount of correction.

Still further, there has been a need for an orthotic which can be readily altered by removing a first wedge and substituting therefor an alternative wedge such as might be necessary to provide a different amount of correction.

Yet further, there has been a need for an orthotic which can be integrated into the sole of an otherwise substantially conventional shoe.

These and other objects of the invention will be better understood from the following Detailed Description of the invention, taken together with the appended Figures.

### The Figures

Figure 1 is a generalized front elevational view of the lower extremities of a patient in need of alignment correction, including an orthotic according to the present invention.

Figure 2a shows a typical orthotic according to the present invention in perspective side view, including front and back correcting wedges.

Figure 2b shows the orthotic of Figure 2a in front elevational view.

Figure 2c shows the orthotic of Figure 2a in bottom view with the correcting wedges removed.

Figure 3 shows in detail an attachment portion of an orthotic according to the present invention.

Figure 4 shows the orthotic attachments of the present invention incorporated into the sole of an otherwise conventional shoe.

Figure 5 shows from rear elevational view one example of the alignment correction available with the adjustable orthotic of the present invention.

**Figures 6A-D show an alternative implementation for attaching a post to an orthotic which has been found especially useful in many situations**.

**Figures 7A-B show an alternative method of attaching posts to an orthotic**.

**Figures 8A-B show an orthotic having a toe extension**.

**Figure 9A-E shows a method of attaching a post involving bolts**.

**Figure 10 shows a shoe having posts affixed by side insertion**.

### Detailed Description of the invention

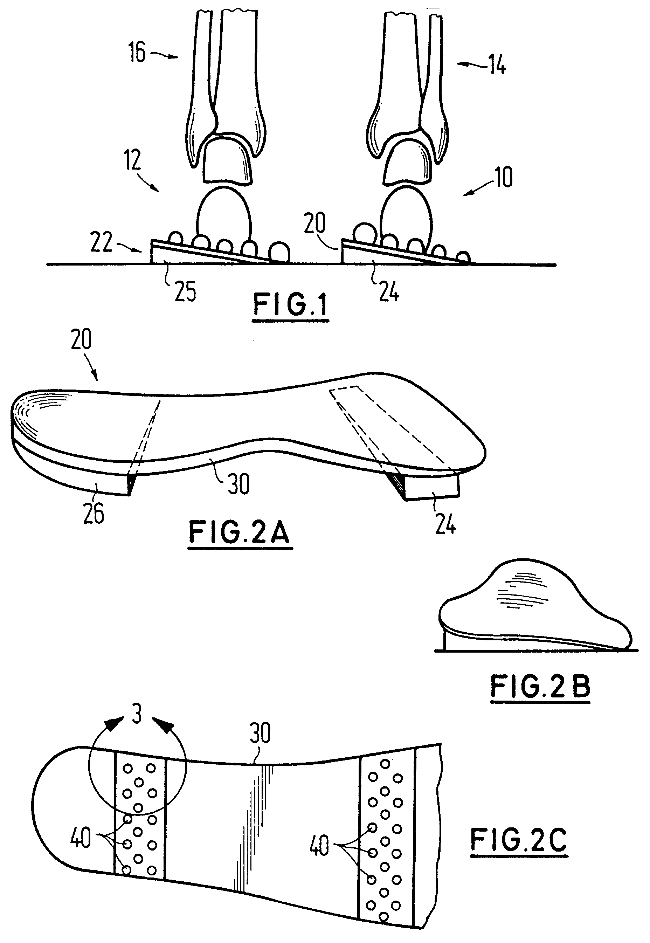

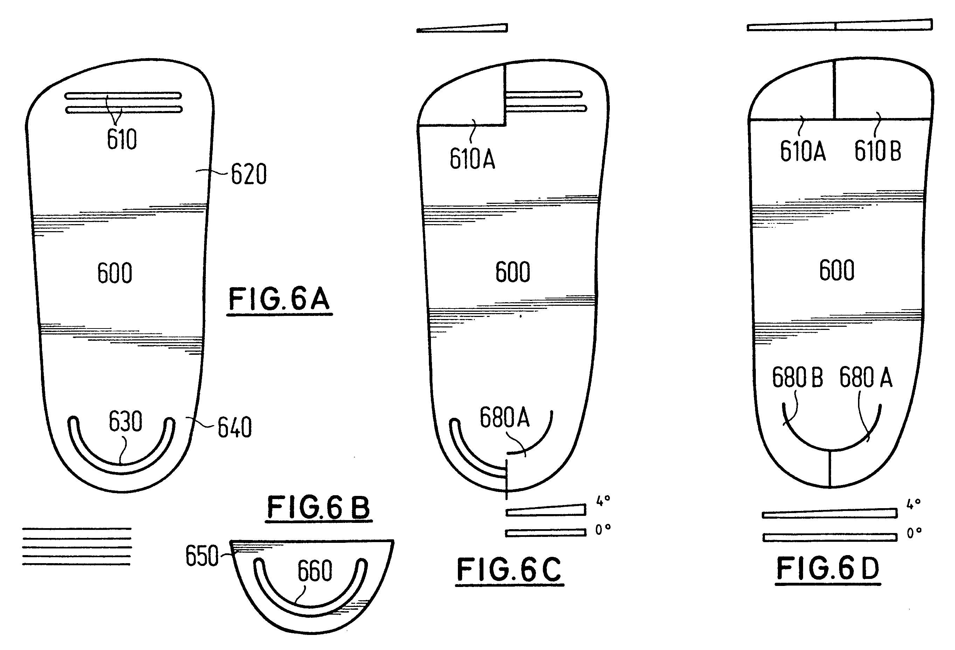

Referring first to Figure 1, the generalized bone structure of the lower extremities of a patient 5 can be seen in front elevational view together with an orthotic according to the present invention. Two feet 10 and 12 support respective legs 14 and 16. As can be seen from Figure 1, the feet can be seen to need correction of alignment relative to the ground and to the remainder of the body. In particular, the right foot 10 is shown in valgus, while the left foot is shown in varus. A pair of orthotic devices, indicated generally at 20 and 22, for left and right feet, respectively, can be seen below the respective feet, with rear foot and forefoot posts or wedges 24 and 26 in place for adjustment of the foot alignment and lower extremity alignment. Those skilled in the art will recognize from Figure 1 that the orthotic of the present invention is intended to be capable of treating both varus and valgus misalignment. With reference to Figure 6, one example of the type of alignment correction available with present invention can be seen. A foot 10, shown in dashed line form within the shoe 17, requires correction. By adding the wedge 26, the plane of the ground is raised to meet the sole of the foot 10, as shown by the line 27. This in turn causes the alignment of the foot 10 to change as shown by the line 29. It will be appreciated that the capability of adding a rear wedge 26 also provides the possibility of adding a heel lift for independent adjustment of heel height.

With reference now to Figures 2a-2c, the orthotic device 20, which for purposes of example only is an orthotic device for the right foot, includes a footsole portion 30 together with the pair of correcting wedges 24 and 26. The wedges 24 and 26 can be seen (Figure 2b) to change the angle of the bottom of the footsole portion 30 relative to the ground, thus bringing the ground up to meet the soles of the patient's foot. It will be appreciated that the wedges 24 and 26 can be formed at any angle, depending on the patient's needs. Likewise, it will be appreciated by those skilled in the art that the orientation of the wedges 24 or 26 can change depending upon whether valgus or varus correction is required. In at least some instances it may be desirable to provide an indicia for readily differentiating wedges of different angles, such as by making wedges of different correction factor in different colors.

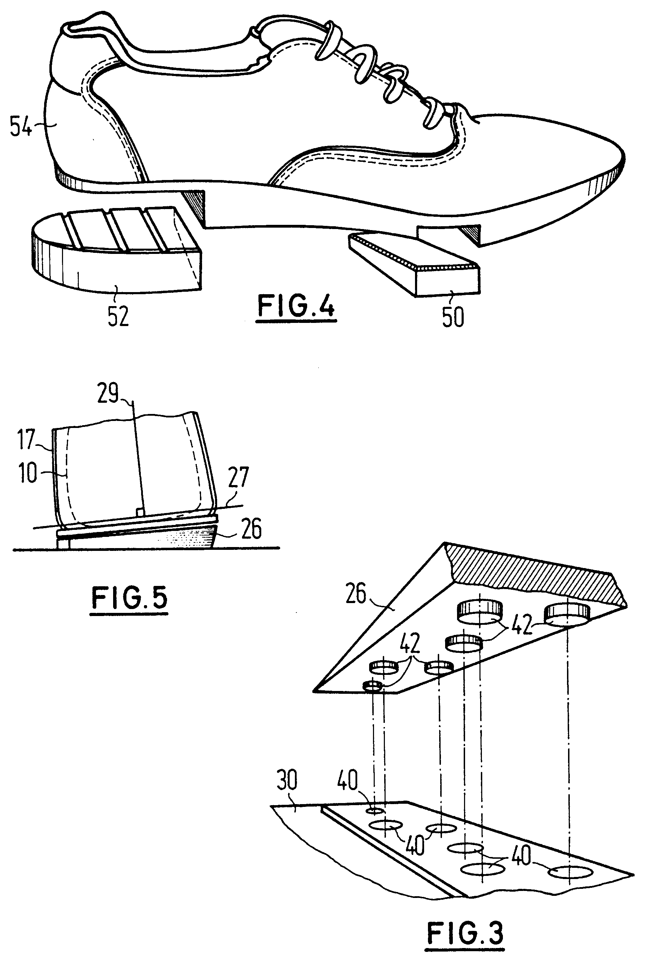

Referring particularly to Figures 2c and 3, a preferred type of attachment device is shown in detail. The bottom of the footsole portion 30 can be seen to have formed therein a pattern of recesses 40, which in the exemplary embodiment shown here are circular but may in other embodiments be of any acceptable form factor. The top portion of the posts or wedges 24 and 26 can also be seen to have a pattern of raised members 42 mated to the pattern of recesses 40, such that the wedges 24 or 26 can be affixed to the footsole portion 30 and will mechanically adhere thereto through the stress of walking, running or other ambulatory movement, while at the same time being readily removable when required for adjustment or other treatment.

Although a pattern of mating recessed and raised members is presently preferred as a method of affixing the wedges 24 or 26 to the footsole 30, other approaches are also acceptable. For example, Velcro™ and the related class of "hook-and-loop" fasteners are acceptable attachment devices in at least some instances, as are T-slot connectors or any other device which provides a firm mechanical attachment which will withstand the stresses of walking or similar activities.

The footsole portion 30 may comprise any of a number of materials, and can be formed by casting, extrusion, or other molding processes. A variety of materials are acceptable, including nylon, polystyrene, high density polyethylene, and numerous other polymers, but the presently preferred material is cast polyethylene terephthalate. For at least some applications, it is preferable for the polyethylene terephthalate to be fiber filled, although fiber content is not necessary in all cases.

The footsole portion 30 may be either preformed or custom molded, depending on the needs of the patient and the economic issues associated with custom molding. In addition, in some embodiments it may also be desirable to make the raised members 42 out of different, more rigid material which may be embedded either in the footsole portion 30 or the orthotic attachments or wedges 24 and 26. Such an arrangement may, depending upon the environment and the materials used for the footsole portion 30 and attachments 24 and 26, provide better retention of the orthotic to the footsole.

Referring next to Figure 4, in some embodiments it may be desirable to integrate orthotic attachments 50 and 52 into the sole of a shoe 54, in which case the footsole portion may be separated from the wedges. Although the shoe of Figure 4 shoes Velcro™ as a means of attachment for purposes of example, the presently preferred method of attachment is the mating raised members and recesses discussed above in connection with Figure 3.

While in many instances it will be desirable to use the same material for the posts as for the footsole, in some instances it may be desirable to vary the materials for the respective portions. For example, improved shock absorption may be provided by using posts of a hard rubber combined with footsole constructed of a thermoplastic material. The easy replacement of the posts also provides an easy method for minimizing normal wear and tear, particularly on the shoe of Figure 5, in that a worn post can simply be removed and a replacement snapped into place.

Additionally, it is possible to provide an adjustable arch support in accordance with the present invention simply by providing a removable arch attachment and providing mating attachment members to a removable arch attachment and to the appropriate portion of the footsole portion 30.

By combining the footsole portion 30 with the adjustable wedges 24 and 26, a completely adjustable, substantially custom fitted orthotic may be provided without the cost or delay normally associated with custom orthotics, while at the same time providing a highly effective method for optimizing balance and support for the patient.

**An alternative method of attaching posts to an orthotic can be appreciated from Figures 6A-6D, in which the bottom an orthotic 600 is shown in Figure 6A. Integrally formed into the orthotic 600 are forefoot grooves 610 at the forefoot portion 620 and rearfoot grooves 630 at the ankle portion 640 of the orthotic 600. One or more such grooves may be provided at each portion, as illustrated in the single rearfoot groove and the double forefoot grooves. A mating rearfoot post 650 shown in Figure 6B, having a ridge 660 formed thereon, connects to the orthotic 600 at the ankle or rearfoot portion 640 thereof by interlocking the ridge 660 into the groove 630. Alternatively, in some instances it is possible that the ridges could be placed on the orthotic and the grooves on the post. As can be appreciated from Figure 6C, a forefoot** post 670A can be provided to mate to the orthotic 600 in a similar manner. The approach of Figure 6C also illustrates that a post need not extend across the entire sole of the orthotic, but instead may extend only part way. Additionally, and as best illustrated in Figures 6C and 6D, a plurality of partial posts may be provided for attachment next to one another, as shown by posts 670A-B and 680A-B. In addition, an arch wedge 690 may be provided if desired.

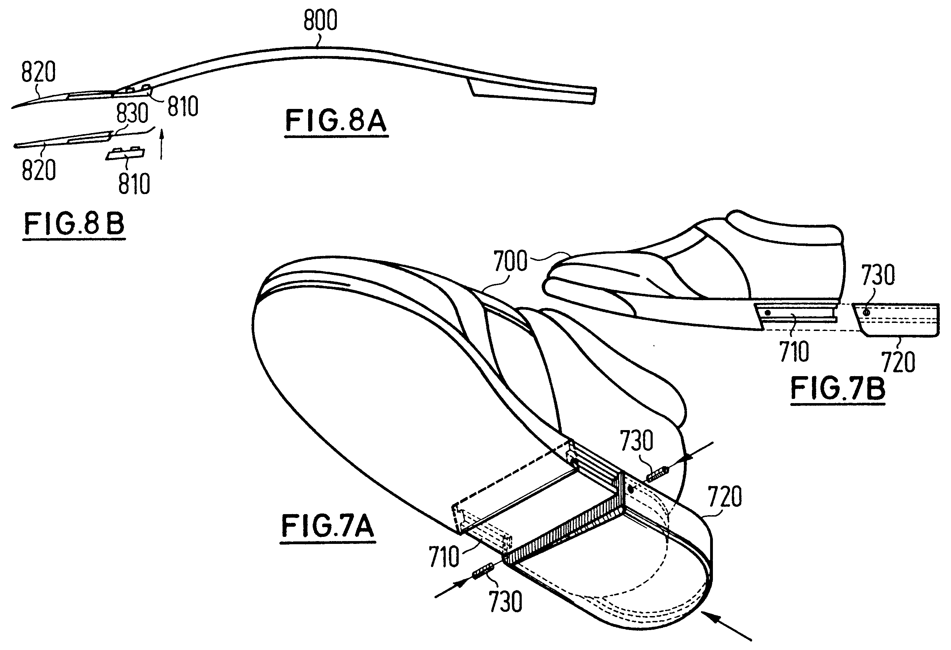

The embodiments of figures 7A-B do not fall within the scope of the claims, but show a further arrangement for affixing a heel post to the shoe. A shoe 700 includes at the heel thereof a mounting portion 710. A heel post 720, which forms substantially the entire heel of the shoe, slides over the mounting portion 710 and is locked into place with one or more fasteners 730, such as screws, dowels or other similar devices. As with other embodiments described above, this approach permits easy replacement of an entire heel, whether as the result of wear or adjustment of the angle of the heel post.

Referring next to Figures 8A-B, an orthotic having a toe extension according to the present invention can be better appreciated. It will be appreciated by those skilled in the art that in some cases support for the toes is required, and yet conventional, customized orthotics are too rigid to permit them to extend into the toe area because of the required flexing of the orthotic at the junction of the toes to the remainder of the foot. In accordance with the present invention, an orthotic 800 having attached thereto a forefoot post 810 mates to a toe extension portion 820 such that a continuous surface is provided between the front of the orthotic 800 and the toe extension 820. The toe extension 820 may be formed integrally with a forefoot post 830, or in some instances and depending upon the material from which the orthotic is made, may be formed integrally with the orthotic. Additionally, the toe extension 820 **may be formed independently of the forefoot post, but configured to be inserted between the attachment devices of the post and the orthotic as shown in Figure 6. Such an arrangement is shown in Figure 8B.**

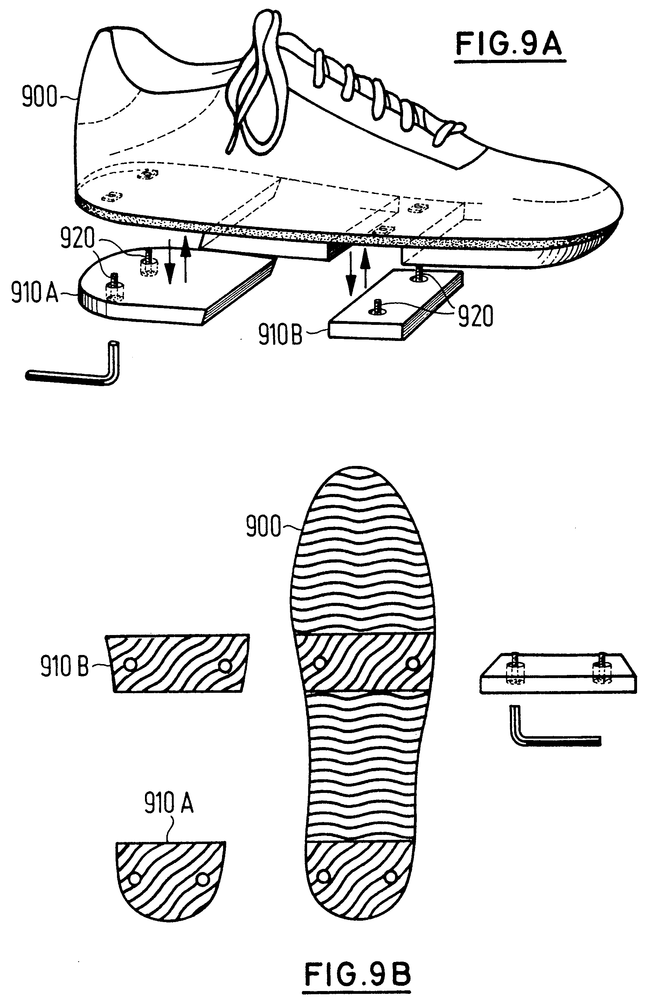

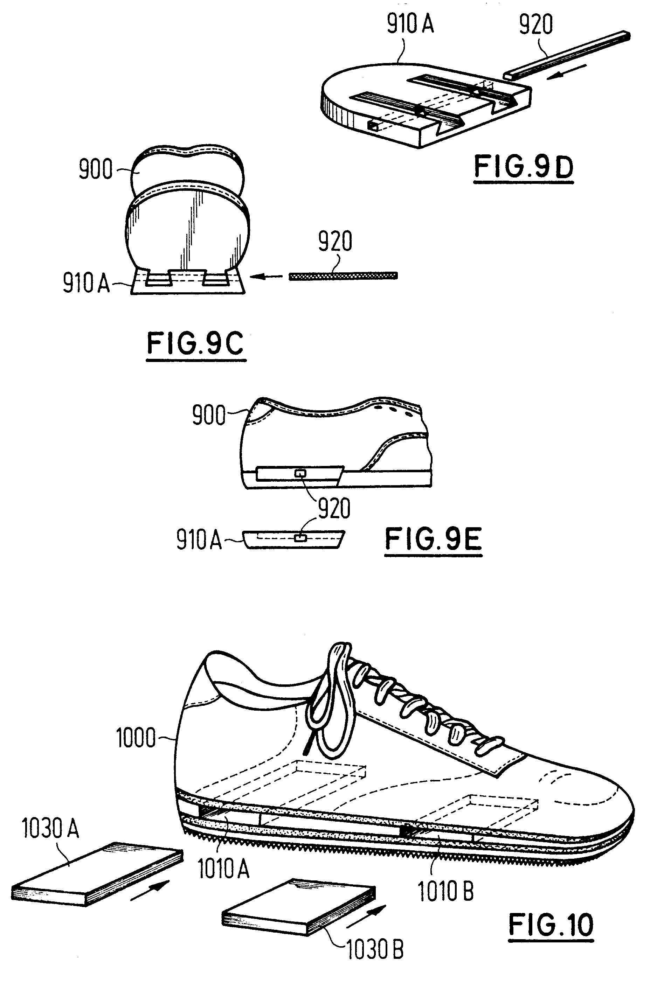

**With reference next to Figure 9A-E, methods for attaching rearfoot and forefoot posts to a shoe using bolts are shown. Figure 9A-B show from the right side and in partial cross-section view a shoe 900 of the type shown in Figure 4, but wherein the posts 910A-B are affixed to the sole of the shoe by bolts or screws 920. Although Allen bolts are shown, any suitable screw or bolt is acceptable.**

**Figures 9C-E still show a further arrangement for affixing a heel post to the shoe (900).**

**Referring next to Figure 10, another approach to affixing corrective posts to a shoe otherwise of the type shown in Figure 4 may be appreciated. In the shoe 1000 shown partial cross-sectional perspective view in Figure 10, slots 1010A and 1010B are provided in the sole 1020, and posts 1030A and 1030B may be inserted into the slots 1010A-B, respectively. Due to the cross-sectional sizing of the posts 1030, the insertion of such posts causes a distortion of the sole of the foot appropriate to the required correction. While the posts 1030A and 1030B are sized sufficiently large that they wedge into place and should remain in place until intentionally removed, if additional security of attachment is desired a dowel or other device may be inserted into the sole 1020 and thence into the respective post 1030 to ensure that the respective post does not inadvertently work its way out of the slot**.

Having fully described one embodiment of the present invention, it will be apparent to those of ordinary skill in the art that numerous alternatives and equivalents exist which do not depart from the invention set forth above. It is therefore to be understood that the invention is not to be limited by the foregoing description, but only by the appended claims.

## Claims

1. An adjustable orthopedic device for the foot comprising a footbed portion (30) formed to conform substantially to the sole of a patient's foot and having first attachment means in a rearfoot area on the bottom thereof, and wedge means (26) having second attachment means on the top thereof, said second attachment means being arranged to mate with the first attachment means to affix the wedge means (26) to the rearfoot area of the footbed portion (30) in such a manner that the wedge means (26) can be removed characterized in that the footbed portion has also first attachment means in a forefoot area on its bottom side which mate with second attachment means on the top of additional removable wedge means (24) in the forefoot area of the footbed portion and that the attachment means are capable of mating with the wedge means (24, 26) for treating varus or valgus misalignments.

2. An adjustable orthopedic device for the foot comprising a shoe (54, 700) having a sole (710), characterized by the sole having first attachment means in a rearfoot area and in a forefoot area on the bottom thereof, and wedge means (24, 26) having second attachment means on the top thereof, the second attachment means being arranged to mate with the first attachment means thereby to affix the wedge means (24, 26) to the rearfoot area and to the forefoot area of the sole of the shoe (54) during walking while at the same time permitting the wedge means (910) to be removed when desired, and whereby the attachment means are capable of mating with the wedge means (910A, 910B) for treating varus or valgus misalignments.

3. The orthopedic device of claim 1 or 2, wherein one of said first and second attachment means comprises raised members (42) and the other of said first and second attachment means comprises recesses (40, 720).

4. The orthopedic device of one of the claims 1 to 3, wherein one of said first and second attachment means comprises a hook portion and the other of said first and second attachment means comprises a loop portion.

5. The orthopedic device of claim 1 to 3, wherein the first attachment means is a groove (610, 630) and the second attachment means is a ridge (660) mated to the groove (610, 630) of the first attachment means.

6. The orthopedic device of claim 1 to 3, wherein the first attachment means is a threaded receiver and the second attachment means is a bolt extending through at least a portion of the wedge means (24, 26).

7. The orthopedic device of claim 2, wherein the first attachment means is a recess within the sole (710) of the shoe (54, 700) and the second attachment means is the perimeter sizing of the wedge means, such that the wedge means is affixed to the sole (710) by being inserted into the recess.

8. The orthopedic device of claim 2, wherein the first attachment means is a slot within the sole of the shoe and the second attachment means is the cross-sectional sizing of the posts.

9. The orthopedic device of claim 1 to 8, wherein the footbed portion (30) is made from polyethylene terephthalate.

10. The orthopedic device of claim 1 to 8, wherein the footbed portion (30) is made from nylon.

11. The orthopedic device of claim 1 to 10, wherein the wedge means (24, 26) are of different colors for different amounts of correction.

12. The orthopedic device of claim 1 or 3 to 6, or 9 to 11, wherein the footbed portion includes an arch wedge.

## Patentansprüche

1. Anpaßbares orthopädisches Gerät für den Fuß, enthaltend einen Fußbettabschnitt (30), der so geformt ist, daß er der Sohle eines Fußes eines Patienten im wesentlichen konform ist, und erste Anbringungsmittel in einem hinteren Fußbereich an seiner Unterseite aufweist, und Keilmittel (26) mit zweiten Anbringungsmitteln an ihrer Oberseite, die derart angeordnet sind, daß sie mit den ersten Anbringungsmitteln zum Anbringen der Keilmittel (26) an dem hinteren Fußbereich des Fußbettabschnittes (30) in solcher Weise zusammenpassen, daß die Keilmittel (26) entfernt werden können, dadurch gekennzeichnet, daß der Fußbettabschnitt auch erste Anbringungsmittel in einem vorderen Fußbereich an seiner Unterseite aufweist, die mit zweiten Anbringungsmitteln auf der Oberseite zusätzlicher Keilmittel (24) in dem vorderen Fußbereich des Fußbettabschnittes zusammenpassen, und daß die Anbringungsmittel in der Lage sind, mit den Kantenmitteln (24, 26) zur Behandlung von Varus- oder Valgus-Fehlstellungen zusammenzupassen.

2. Einstellbares orthopädisches Gerät für den Fuß, enthaltend einen Schuh (54, 700) mit einer Sohle (710), dadurch gekennzeichnet, daß die Sohle erste Anbringungsmittel in einem hinteren Fußbereich und in einem vorderen Fußbereich an ihrer Unterseite aufweist, und gekennzeichnet durch Keilmittel (24, 26) mit zweiten Anbringungsmitteln auf ihrer Oberseite, wobei die zweiten Anbringungsmittel so angeordnet sind, daß sie mit den ersten Anbringungsmitteln zusammenpassen zur Anbringung der Keilmittel (24, 26) an den hinteren Fußbereich und den vorderen Fußbereich der Sohle des Schuhs (54) während des Gehens, während sie gleichzeitig zulassen, daß die Keilmittel (910) nach Wunsch entfernt werden können, und wodurch die Anbringungsmittel in der Lage sind, mit den Keilmitteln (910A, 910B) zur Behandlung von Varus- oder Valgus-Fehlstellungen zusammenzupassen.

3. Orthopädisches Gerät nach Anspruch 1 oder 2, wobei eines der ersten und zweiten Anbringungsmittel erhabene Elemente (42) und das jeweils andere der Anbringungsmittel Ausnehmungen (40, 720) umfaßt.

4. Orthopädisches Gerät nach einem der Ansprüche 1 bis 3, wobei eines der ersten und zweiten Anbringungsmittel einen Hakenabschnitt und das jeweils andere der Anbringungsmittel einen Schleifenabschnitt umfaßt.

5. Orthopädisches Gerät nach Anspruch 1 bis 3, wobei das erste Anbringungsmittel eine Nut (610, 630) und das zweite Anbringungsmittel eine Rippe (660) ist, die an die Nut (610, 630) des ersten Anbringungsmittels angepaßt ist.

6. Orthopädisches Gerät nach Anspruch 1 bis 3, wobei das erste Anbringungsmittel eine Gewindeaufnahme und das zweite Anbringungsmittel ein sich durch mindestens einen Teil der Keilmittel (24, 26) erstreckender Bolzen ist.

7. Orthopädisches Gerät nach Anspruch 2, wobei das erste Anbringungsmittel eine Ausnehmung in der Sohle (710) des Schuhs (54, 700) und das zweite Anbringungsmittel die Umfangsabmessung der Keilmittel ist, derart, daß das Keilmittel an der Sohle (710) durch Einsetzen in die Ausnehmung befestigt wird.

8. Orthopädisches Gerät nach Anspruch 2, wobei das erste Anbringungsmittel ein Schlitz in der Sohle des Schuhs und das zweite Anbringungsmittel die Querschnittsgrößenauswahl der Pfosten ist.

9. Orthopädisches Gerät nach Anspruch 1 bis 8, wobei der Fußbettabschnitt (30) aus Polyethylenterephthalat hergestellt ist.

10. Orthopädisches Gerät nach Anspruch 1 bis 8, bei dem der Fußbettabschnitt (30) aus Nylon besteht.

11. Orthopädisches Gerät nach Anspruch 1 bis 10, wobei die Keilmittel (24, 26) für unterschiedliche Korrekturgrößen unterschiedliche Farbe aufweisen.

12. Orthopädisches Gerät nach Anspruch 1 oder 3 bis 6 oder 9 bis 11, wobei der Fußbettabschnitt einen Fußgewölbekeil enthält.

## Revendications

1. Dispositif orthopédique ajustable pour le pied, comprenant une partie de semelle (30) formée de façon à se conformer pratiquement à la plante du pied d'un patient et comportant des premiers moyens de fixation dans une zone de talon sur le dessous de celle-ci, et un moyen de coin (26) comportant des seconds moyens de fixation sur le dessus de celui-ci, lesdits seconds moyens de fixation étant agencés pour s'accoupler avec les premiers moyens de fixation afin de fixer le moyen de coin (26) à la zone de talon de la partie de semelle (30) d'une manière telle que le moyen de coin (26) puisse être enlevé, caractérisé en ce que la partie de semelle comporte également des premiers moyens de fixation dans une zone antérieure du pied sur son dessous, qui s'accouplent avec des seconds moyens de fixation sur le dessus d'un moyen de coin amovible supplémentaire (24) dans la zone antérieure du pied de la partie de semelle et en ce que les moyens de fixation peuvent s'adapter aux moyens de coin (24, 26) afin de traiter des défauts d'alignement du type varus ou valgus.

2. Dispositif orthopédique ajustable pour le pied, comprenant une chaussure (54, 700) comportant une semelle (710), caractérisé ce que la semelle comporte des premiers moyens de fixation dans une zone de talon et dans une zone antérieure du pied sur son dessous, et des moyens de coin (24, 26) comportant des seconds moyens de fixation sur leur dessus, les seconds moyens de fixation étant agencés pour s'accoupler avec les premiers moyens de fixation afin de fixer ainsi les moyens de coin (24, 26) à la zone du talon et à la zone antérieure du pied de la semelle de la chaussure (54) pendant la marche tout en permettant en même temps que les moyens de coin (910) soient enlevés lorsqu'on le souhaite, et d'où il résulte que les moyens de fixation peuvent s'adapter aux moyens de coin (910A, 910B) afin de traiter des défauts d'alignement du type varus ou valgus.

3. Dispositif orthopédique selon la revendication 1 ou la revendication 2, dans lequel l'un desdits premier et second moyens de fixation comprend des éléments surélevés (42) et l'autre desdits premier et second moyens de fixation comprend des évidements (40, 720).

4. Dispositif orthopédique selon l'une des revendications 1 à 3, dans lequel l'un desdits premier et second moyens de fixation comprend une partie de crochets et l'autre desdits premier et second moyens de fixation comprend une partie de boucles.

5. Dispositif orthopédique selon les revendications 1 à 3, dans lequel le premier moyen de fixation est une rainure (610, 630) et le second moyen de fixation est une nervure (660) adaptée à la rainure (610, 630) du premier moyen de fixation.

6. Dispositif orthopédique selon les revendications 1 à 3, dans lequel le premier moyen de fixation est un alésage fileté et le second moyen de fixation est un boulon s'étendant au travers d'au moins une partie des moyens de coin (24, 26).

7. Dispositif orthopédique selon la revendication 2, dans lequel le premier moyen de fixation est un évidement à l'intérieur de la semelle (710) de la chaussure (54, 700) et le un second moyen de fixation est le dimensionnement périmétrique des moyens de coin, de sorte que les moyens de coin sont fixés à la semelle (710) en étant insérés dans l'évidement.

8. Dispositif orthopédique selon la revendication 2, dans lequel le premier moyen de fixation est une fente à l'intérieur de la semelle de la chaussure et le second moyen de fixation est le dimensionnement en section transversale des tenons.

9. Dispositif orthopédique selon les revendications 1 à 8, dans lequel la partie de semelle (30) est faite de téréphtalate de polyéthylène.

10. Dispositif orthopédique selon les revendications 1 à 8, dans lequel la partie de semelle (30) est faite de nylon.

11. Dispositif orthopédique selon les revendications 1 à 10, dans lequel les moyens de coin (24, 26) sont de différentes couleurs pour différentes valeurs de correction.

12. Dispositif orthopédique selon la revendication 1, ou 3 à 6, ou 9 à 11, dans lequel la partie de semelle comprend un support de voûte plantaire.
